# EUROPEAN PATENT APPLICATION

(11) **EP 4 368 017 A1**
(43) Date of publication of application: **15.05.2024**
(21) Application number: 22398023.6
(22) Date of filing: 09.11.2022
(51) Int. Cl.: A01H 7/00, C12Q 1/6895

(54) **METHOD FOR GENETIC SELECTION BASED ON PINE CONE PRODUCTION**

(71) Applicant: Cebal - Centro De Biotecnologia Agrícola E Agro-Alimentar Do Alentejo, 7801-908 Beja (PT)
(72) Inventor: Usié Chimenos, Ana Isabel, 7800-295 Beja (PT); Fialho Leão, Célia Cristina, 2780-155 Oeiras (PT); Pinheiro Ribeiro de Meireles, Brígida da Natividade, 2685-019 Sacavém (PT); Mendes, Bruna Lopes, 5070-050 Alijó (PT); Costa do Amaral Ramos, António Marcos, 3534-004 Mangualde (PT); Alves Antunes, Marta Maria, 7800-295 Beja (PT)
(74) Representative: Couto, Cláudia

(57) **Abstract**

The present patent application discloses a method for the genetic selection of trees from the stone pine species *(Pinus pinea*) with a genetic profile associated with higher pinecone production. Said profile is determined based on a group of Single Nucleotide Polymorphisms (SNPs) herein associated with higher pinecone production in the stone pine.

## Description

### Technical field

The present patent application relates to a method for the genetic selection of trees from the stone pine species based on pinecone production.

### Background art

Stone pine (*Pinus pinea*) is a Mediterranean species with settlements located from the western cost of the Iberian Peninsula to Turkey with an area of about 650,000 ha. Between 1995 and 2010 the Portuguese stone pine area increased by 46% reaching now 175,000 ha. The main interest in this species in Portugal is the seed production: pine nuts or pine kernels. The cone needs three growing seasons to complete its maturation. The first cones begin to appear on trees with about 10 years old, but harvest profitable productivity starts around 20 years reaching maximum productivity between 40 and 60 years. The number of cones produced per tree varies between regions averaging 100-120 cones per tree. The main constraint identified by pine kernel and pinecone production is the early identification of plants with good yield for pine kernel production.

To date, association studies in conifers in order to identify genetic markers have had, so far, some limitations due to its large genome and scarce genetic resources, which implied a high cost to genotype the number of individuals. However, some association studies were successfully conducted in conifers such as *Pinus tadea* using Single Nucleotide Polymorphisms (SNPs) characterized in studies of candidate genes or Expressed Sequence Tags (ESTs), for phenotypes of interest, offering the potential for determining the variation present in each individual analysed, despite its low genetic diversity. When this process is applied to groups of individuals with contrasting phenotypic characteristics it is possible to identify the genetic variations most different among each group of individuals. In this way, is then possible to identify the most relevant SNPs that can later be used to select individuals carrying the variants of greatest interest.

### Summary

The present application is related to a method for the genetic selection of trees from stone pine based on pinecone production characterized by comprising the following steps:
- Preparation of a stone pine tree biological sample and extraction of DNA from said sample;
- Genotyping the DNA sample for at least one Single Nucleotide Polymorphisms selected from the sequences SEQ ID NO: 1 to 18 associated with higher pinecone production;
- Determining the stone pine tree's production based on the presence of at least one of said SNPs of sequences SEQ ID NO: 1 to 18 associated with higher pinecone production.

In one embodiment the sample is a sample of needles.

The present application also relates to Single Nucleotide Polymorphisms of sequences SEQ ID NO: 1 to 18 for use as biomarkers of pinecone production in stone pine trees.

### General description

The present patent application relates to a method for the genetic selection of trees from the stone pine species based on pinecone.

The method for the genetic selection of trees from the stone pine species based on pinecone production and on genotypes associated with higher pinecone production comprises the following steps:
• Preparation of a stone pine tree biological sample and extraction of DNA from said sample;
• Genotyping the DNA sample for at least one of the Single Nucleotide Polymorphisms (SNPs) of Table 1:

**Table 1. SNPs used in the present application**

| **#SNP_ID** | **G1_F A0** | **G1_F A1** | **G2_F A0** | **G2_F A1** | **Differ ence** | **Gene** |
|---|---|---|---|---|---|---|
| PP_SNP1.1 and 1.2 | 0.40 | 0.60 | 1.00 | 0.00 | 0.60 | Transcription elongation factor SPT6 |
| PP_SNP2.1 and 2.2 | 0.20 | 0.80 | 0.90 | 0.10 | 0.70 | GDP-mannose 3,5-epimerase |
| PP_SNP3.1 and 3.2 | 0.17 | 0.83 | 0.75 | 0.25 | 0.58 | 1-aminocycloprop ane-1-carboxylate oxidase |
| PP_SNP4.1 and 4.2 | 0.59 | 0.41 | 0.04 | 0.96 | 0.55 | Tryptophan aminotransfera se-related protein 3 |
| PP_SNP5.1 and 5.2 | 0.62 | 0.38 | 0.00 | 1.00 | 0.62 | Ultraviolet-B receptor UVR8 |
| PP_SNP6.1 and 6.2 | 0.10 | 0.90 | 0.85 | 0.15 | 0.75 | Cyclin-dependent kinase F-1 |
| PP_SNP7.1 and 7.2 | 0.05 | 0.95 | 0.88 | 0.12 | 0.83 | Purple acid phosphatase 4 |
| PP_SNP8.1 and 8.2 | 0.00 | 1.00 | 0.92 | 0.08 | 0.92 | Lleucine-rich repeat receptor-like protein kinase |
| PP_SNP9.1 and 9.2 | 0.84 | 0.16 | 0.72 | 0.28 | 0.12 | E3 ubiquitin-protein ligase XBOS34 |

• Determining the stone pine tree's production based on the presence of at least one of said SNPs significantly associated with higher pinecone production.

### Detailed description of embodiments

Now, preferred embodiments of the present application will be described in detail with reference to the annexed drawings. However, they are not intended to limit the scope of this application.

The present patent application discloses a method for the genetic selection of trees from the stone pine species with a profile associated with higher pinecone production. Said profile is determined based on a group of SNPs disclosed herein associated with a higher pinecone production.

The technology disclosed herein is based on the sequencing of the transcriptome of stone pine trees, to identify the global set of variations, namely SNPs, present in the codifying regions of each tree. This sequencing is done on groups of individuals with contrasting phenotypes of pinecone production. The next step involves determining the allele frequencies observed in each of the groups and identifying the SNPs with the largest differences observed between the groups for the frequency of the alternative allele. Once the SNPs with the greatest difference in allele frequency were identified, it became possible to genotype these SNPs in other trees, selecting the trees carrying the most frequent alleles in the group of trees, that showed the best performances for pinecone production.

Nine SNPs are identified herein and associated with an improvement in pinecone production in stone pine trees. The complete list of these SNPs comprises two sequence variations per SNP, which results in eighteen sequences, as shown in Table 2.

**Table 2. List of SNP sequences associated with a higher pinecone production**

| **#SNP_ID** | **SEQ ID NO** | **Sequence** | **Gene** |
|---|---|---|---|
| PP_SNP1.1 | SEQ ID NO: 1 | AAGTGGATTTTGCTTCAAAGGAG AAAGAATGCTCTTAAAAACATCT TATGAGAAGCGTGCTGCAGAAGA AGCACAGAGAGA | Transcri ption elongati on factor SPT6 |
| PP_SNP1.2 | SEQ ID NO: 2 | AAGTGGATTTTGCTTCAAAGGAG AAAGAATGCTCTTAAAAGCATCT TATGAGAAGCGTGCTGCAGAAGA AGCACAGAGAGA | |
| PP_SNP2.1 | SEQ ID NO: 3 | ACTTTTTATTCTCAAAGCTTGAC ACCATTTCAGCCATCTCGTTCAT GCTGACCATTTCATCACTGCCAA TATTCACTGGCT | GDP-mannose 3,5-epimeras e |
| PP_SNP2.2 | SEQ ID NO: 4 | ACTTTTTATTCTCAAAGCTTGAC ACCATTTCAGCCATCTCATTCAT GCTGACCATTTCATCACTGCCAA TATTCACTGGCT | |
| PP_SNP3.1 | SEQ ID NO: 5 | GGGCAAGGAGGGTAATAATTGAT TCTGACTTGTAGCAGGGGTTCCT TATTGAGGGTTTGGATATACTTT GGGTCCAGCCCC | 1-aminocyc lopropan e-1-carboxyl ate oxidase |
| PP_SNP3.2 | SEQ ID NO: 6 | GGGCAAGGAGGGTAATAATTGAT TCTGACTTGTAGCAGGGCTTCCT TATTGAGGGTTTGGATATACTTT GGGTCCAGCCCC | |
| PP_SNP4.1 | SEQ ID NO: 7 | CTAACAACGCGAAATTGTCGTCC CGTCTGATCAAGCTCAATCGAAC ATACCTTTTCTCTCCTCCGAACT CTGGGCCTCCTC | Tryptoph an aminotra nsferase -related protein 3 |
| PP_SNP4.2 | SEQ ID NO: 8 | CTAACAACGCGAAATTGTCGTCC CGTCTGATCAAGCTCAACCGAAC ATACCTTTTCTCTCCTCCGAACT CTGGGCCTCCTC | |
| PP_SNP5.1 | SEQ ID NO: 9 | AGAGGAAGACCTTTGTTCTCTCT GATGATGGGTTGGTATTTGCTTT TGGATGGATGGCTTTTGGAAGCT TGGGATTTGTGA | Ultravio let-B receptor UVR8 |
| PP_SNP5.2 | SEQ ID NO: 10 | AGAGGAAGACCTTTGTTCTCTCT GATGATGGGTTGGTATTCGCTTT TGGATGGATGGCTTTTGGAAGCT TGGGATTTGTGA | |
| PP_SNP6.1 | SEQ ID NO: 11 | GGCAGAAACTGCGAATTTGGATT TGAGCTGGAAAAATGAAGGTGAT ATGGTGATGCAGGGATTCACATC TGGTGTTGGAAC | Cyclin-dependen t kinase F-1 |
| PP_SNP6.2 | SEQ ID NO: 12 | GGCAGAAACTGCGAATTTGGATT TGAGCTGGAAAAATGAAAGTGAT ATGGTGATGCAGGGATTCACATC TGGTGTTGGAAC | |
| PP_SNP7.1 | SEQ ID NO: 13 | GATCCAGCTGGGCCAAAACGTCC CCTCTATAGTCATGGTTGCCTAA AACAGCATACCAAGGTTTTTGTA GACTCTTTGCAG | Purple acid phosphat ase 4 |
| PP_SNP7.2 | SEQ ID NO: 14 | GATCCAGCTGGGCCAAAACGTCC CCTCTATAGTCATGGTTACCTAA AACAGCATACCAAGGTTTTTGTA GACTCTTTGCAG | |
| PP_SNP8.1 | SEQ ID NO: 15 | ATATCTCCACTTTGAATGACTTG CAAATTTYCACSAACCACCTCAC TGGCAGCATTCCACAAGAACTGG GCCTTCTCACCC | Lleucine -rich repeat receptor -like protein kinase |
| PP_SNP8.2 | SEQ ID NO: 16 | ATATCTCCACTTTGAATGACTTG CAAATTTYCACSAACCATCTCAC TGGCAGCATTCCACAAGAACTGG GCCTTCTCACCC | |
| PP_SNP9.1 | SEQ ID NO: 17 | CAGCTGCTGGCACAGCAACAGAA TTTGCAGAAACTGATGGGCTAGA AACTGTGGGATCATTTGTCAGAG TTTCAGCAGTGC | E3 ubiquiti n-protein ligase XBOS34 |
| PP_SNP9.2 | SEQ ID NO: 18 | CAGCTGCTGGCACAGCAACAGAA TTTGCAGAAACTGATGGCCTAGA AACTGTGGGATCATTTGTCAGAG TTTCAGCAGTGC | |

The method of the present patent application involves several steps. Initially, it is necessary to collect a sample of biological material, such as a needle sample, for DNA extraction. Next, this DNA sample is genotyped for the SNPs of interest, and the genotypes obtained for each SNP are evaluated. The decision to select the tree is based on the presence of the genotype(s) significantly associated with higher pinecone production.

The method for the genetic selection of trees from the stone pine species based on pinecone production comprises the following steps:
- Preparation of a stone pine tree biological sample and extraction of DNA from said sample;
- Genotyping the DNA sample for at least one Single Nucleotide Polymorphisms selected from the sequences SEQ ID NO: 1 to 18 associated with higher pinecone production;
- Determining the stone pine tree's production based on the presence of at least one of said SNPs of sequences SEQ ID NO: 1 to 18 associated with higher pinecone production.

In one embodiment, the sample analysed is preferably a sample of needles.

In another embodiment, the SNPs of sequences SEQ ID NO: 1 to 18 are used as biomarkers of pinecone production in stone pine trees, i.e., higher production.

**Sequence Listing**

| **1** | **Sequence Listing Information** | |
|---|---|---|
| 1-1 | File Name | 20220824 SelectPinea_Sequence List.xml |
| 1-2 | DTD Version | V1_3 |
| 1-3 | Software Name | WIPO Sequence |
| 1-4 | Software Version | 2.1.2 |
| 1-5 | Production Date | 2022-08-24 |
| 1-6 | Original free text language code | en |
| 1-7 | Non English free text language code | |

| **2** | **General Information** | |
|---|---|---|
| 2-1 | Current application: IP Office | EP |
| 2-2 | Current application: Application number | N/A |
| 2-3 | Current application: Filing date | |
| 2-4 | Current application: Applicant file reference | 202142297 |
| 2-5 | Earliest priority application: IP Office | |
| 2-6 | Earliest priority application: Application number | |
| 2-7 | Earliest priority application: Filing date | |
| 2- 8en | Applicant name | CEBAL - Centro de Biotecnologia Agricola e Agro-alimentar do Alentejo |
| 2- 8en | Applicant name: Name Latin | no |
| 2-9 | Inventor name | |
| 2- 9en | Inventor name: Name Latin | |
| 2-10en | Invention title | METHOD FOR GENETIC SELECTION OF TREES FROM THE STONE PINE SPECIES BASED ON PINECONE PRODUCTION |
| 2-11 | Sequence Total Quantity | 18 |

| **3-1** | **Sequences** | |
|---|---|---|
| 3-1-1 | Sequence Number [ID] | 1 |
| 3-1-2 | Molecule Type | RNA |
| 3-1-3 | Length | 81 |
| 3-1-4-1 | Features Location/Qualifiers | **source** 1..81 mol_type= transcribed RNA organism= Pinus pinea |
| | NonEnglishQualifier Value | |
| 3-1-5 | Residues | aagtggattt tgcttcaaag gagaaagaat gctcttaaaa acatcttatg agaagcgtgc 60 |
| | | tgcagaagaa gcacagagag a 81 |

| **3-2** | **Sequences** | |
|---|---|---|
| 3-2-1 | Sequence Number [ID] | 2 |
| 3-2-2 | Molecule Type | RNA |
| 3-2-3 | Length | 81 |
| 3-2-4-1 | Features Location/Qualifiers | **source** 1..81 mol_type= transcribed RNA organism= Pinus pinea |
| | NonEnglishQualifier Value | |
| 3-2-5 | Residues | aagtggattt tgcttcaaag gagaaagaat gctcttaaaa gcatcttatg agaagcgtgc 60 tgcagaagaa gcacagagag a 81 |

| **3-3** | **Sequences** | |
|---|---|---|
| 3-3-1 | Sequence Number [ID] | 3 |
| 3-3-2 | Molecule Type | RNA |
| 3-3-3 | Length | 81 |
| 3-3-4-1 | Features Location/Qualifiers | **source** 1..81 mol_type= transcribed RNA organism= Pinus pinea |
| | NonEnglishQualifier Value | |
| 3-3-5 | Residues | actttttatt ctcaaagctt gacaccattt cagccatctc gttcatgctg accatttcat 60 cactgccaat attcactggc t 81 |

| **3-4** | **Sequences** | |
|---|---|---|
| 3-4-1 | Sequence Number [ID] | 4 |
| 3-4-2 | Molecule Type | RNA |
| 3-4-3 | Length | 81 |
| 3-4-4-1 | Features Location/Qualifiers | **source** 1..81 mol_type= transcribed RNA organism= Pinus pinea |
| | NonEnglishQualifier Value | |
| 3-4-5 | Residues | actttttatt ctcaaagctt gacaccattt cagccatctc attcatgctg accatttcat 60 cactgccaat attcactggc t 81 |

| **3-5** | **Sequences** | |
|---|---|---|
| 3-5-1 | Sequence Number [ID] | 5 |
| 3-5-2 | Molecule Type | RNA |
| 3-5-3 | Length | 81 |
| 3-5-4-1 | Features Location/Qualifiers | **source** 1..81 mol_type= transcribed RNA organism= Pinus pinea |
| | NonEnglishQualifier Value | |
| 3-5-5 | Residues | gggcaaggag ggtaataatt gattctgact tgtagcaggg gttccttatt gagggtttgg 60 atatactttg ggtccagccc c 81 |

| **3-6** | **Sequences** | |
|---|---|---|
| 3-6-1 | Sequence Number [ID] | 6 |
| 3-6-2 | Molecule Type | RNA |
| 3-6-3 | Length | 81 |
| 3-6-4-1 | Features Location/Qualifiers | **source** 1..81 mol_type= transcribed RNA organism= Pinus pinea |
| | NonEnglishQualifier Value | |
| 3-6-5 | Residues | gggcaaggag ggtaataatt gattctgact tgtagcaggg cttccttatt gagggtttgg 60 atatactttg ggtccagccc c 81 |

| **3-7** | **Sequences** | |
|---|---|---|
| 3-7-1 | Sequence Number [ID] | 7 |
| 3-7-2 | Molecule Type | RNA |
| 3-7-3 | Length | 81 |
| 3-7-4-1 | Features Location/Qualifiers | **source** 1..81 mol_type= transcribed RNA organism= Pinus pinea |
| | NonEnglishQualifier Value | |
| 3-7-5 | Residues | ctaacaacgc gaaattgtcg tcccgtctga tcaagctcaa tcgaacatac cttttctctc 60 ctccgaactc tgggcctcct c 81 |

| **3-8** | **Sequences** | |
|---|---|---|
| 3-8-1 | Sequence Number [ID] | 8 |
| 3-8-2 | Molecule Type | RNA |
| 3-8-3 | Length | 81 |
| 3-8-4-1 | Features Location/Qualifiers | **source** 1..81 mol_type= transcribed RNA organism= Pinus pinea |
| | NonEnglishQualifier Value | |
| 3-8-5 | Residues | ctaacaacgc gaaattgtcg tcccgtctga tcaagctcaa ccgaacatac cttttctctc 60 ctccgaactc tgggcctcct c 81 |

| **3-9** | **Sequences** | |
|---|---|---|
| 3-9-1 | Sequence Number [ID] | 9 |
| 3-9-2 | Molecule Type | RNA |
| 3-9-3 | Length | 81 |
| 3-9-4-1 | Features Location/Qualifiers | **source** 1..81 mol_type= transcribed RNA organism= Pinus pinea |
| | NonEnglishQualifier Value | |
| 3-9- 5 | Residues | agaggaagac ctttgttctc tctgatgatg ggttggtatt tgcttttgga tggatggctt 60 ttggaagctt gggatttgtg a 81 |

| **3-10** | **Sequences** | |
|---|---|---|
| 3-10-1 | Sequence Number [ID] | 10 |
| 3- | Molecule Type | RNA |
| 10-2 | | |
| 3-10-3 | Length | 81 |
| 3-10-4-1 | Features Location/Qualifiers | **source** 1..81 mol_type= transcribed RNA organism= Pinus pinea |
| | NonEnglishQualifier Value | |
| 3-10-5 | Residues | agaggaagac ctttgttctc tctgatgatg ggttggtatt cgcttttgga tggatggctt 60 ttggaagctt gggatttgtg a 81 |

| **3-11** | **Sequences** | |
|---|---|---|
| 3-11-1 | Sequence Number [ID] | 11 |
| 3-11-2 | Molecule Type | RNA |
| 3-11-3 | Length | 81 |
| 3-11-4-1 | Features Location/Qualifiers | **source** 1..81 mol_type= transcribed RNA organism= Pinus pinea |
| | NonEnglishQualifier Value | |
| 3-11-5 | Residues | ggcagaaact gcgaatttgg atttgagctg gaaaaatgaa ggtgatatgg tgatgcaggg 60 attcacatct ggtgttggaa c 81 |

| **3-12** | **Sequences** | |
|---|---|---|
| 3-12-1 | Sequence Number [ID] | 12 |
| 3-12-2 | Molecule Type | RNA |
| 3-12-3 | Length | 81 |
| 3-12-4-1 | Features Location/Qualifiers | **source** 1..81 mol_type= transcribed RNA organism= Pinus pinea |
| | NonEnglishQualifier Value | |
| 3-12-5 | Residues | ggcagaaact gcgaatttgg atttgagctg gaaaaatgaa agtgatatgg tgatgcaggg 60 attcacatct ggtgttggaa c 81 |

| **3-13** | **Sequences** | |
|---|---|---|
| 3-13-1 | Sequence Number [ID] | 13 |
| 3-13-2 | Molecule Type | RNA |
| 3-13-3 | Length | 81 |
| 3-13-4-1 | Features Location/Qualifiers | **source** 1..81 mol_type= transcribed RNA organism= Pinus pinea |
| | NonEnglishQualifier Value | |
| 3-13-5 | Residues | gatccagctg ggccaaaacg tcccctctat agtcatggtt gcctaaaaca gcataccaag 60 gtttttgtag actctttgca g 81 |

| **3-14** | **Sequences** | |
|---|---|---|
| 3-14-1 | Sequence Number [ID] | 14 |
| 3-14-2 | Molecule Type | RNA |
| 3-14-3 | Length | 81 |
| 3-14-4-1 | Features Location/Qualifiers | **source** 1..81 mol_type= transcribed RNA organism= Pinus pinea |
| | NonEnglishQualifier Value | |
| 3-14-5 | Residues | gatccagctg ggccaaaacg tcccctctat agtcatggtt acctaaaaca gcataccaag 60 gtttttgtag actctttgca g 81 |

| **3-15** | **Sequences** | |
|---|---|---|
| 3-15-1 | Sequence Number [ID] | 15 |
| 3-15-2 | Molecule Type | RNA |
| 3-15-3 | Length | 81 |
| 3-15-4-1 | Features Location/Qualifiers | **source** 1..81 mol_type= transcribed RNA organism= Pinus pinea |
| | NonEnglishQualifier Value | |
| 3-15-5 | Residues | atatctccac tttgaatgac ttgcaaattt ycacsaacca cctcactggc agcattccac 60 aagaactggg ccttctcacc c 81 |

| **3-16** | **Sequences** | |
|---|---|---|
| 3-16-1 | Sequence Number [ID] | 16 |
| 3-16-2 | Molecule Type | RNA |
| 3-16-3 | Length | 81 |
| 3-16-4-1 | Features Location/Qualifiers | **source** 1..81 mol_type= transcribed RNA organism= Pinus pinea |
| | NonEnglishQualifier Value | |
| 3-16-5 | Residues | atatctccac tttgaatgac ttgcaaattt ycacsaacca tctcactggc agcattccac 60 aagaactggg ccttctcacc c 81 |

| **3-17** | **Sequences** | |
|---|---|---|
| 3-17-1 | Sequence Number [ID] | 17 |
| 3-17-2 | Molecule Type | RNA |
| 3-17-3 | Length | 81 |
| 3-17-4-1 | Features Location/Qualifiers | **source** 1..81 mol_type= transcribed RNA organism= Pinus pinea |
| | NonEnglishQualifier Value | |
| 3-17-5 | Residues | cagctgctgg cacagcaaca gaatttgcag aaactgatgg gctagaaact gtgggatcat 60 ttgtcagagt ttcagcagtg c 81 |

| **3-18** | **Sequences** | |
|---|---|---|
| 3-18-1 | Sequence Number [ID] | 18 |
| 3-18-2 | Molecule Type | RNA |
| 3-18-3 | Length | 81 |
| 3-18-4-1 | Features Location/Qualitiers | **source** 1..81 mol_type= transcribed RNA organism= Pinus pinea |
| | NonEnglishQualifier Value | |
| 3-18-5 | Residues | cagctgctgg cacagcaaca gaatttgcag aaactgatgg cctagaaact gtgggatcat 60 ttgtcagagt ttcagcagtg c 81 |

## Claims

1. Method for the genetic selection of trees from stone pine based on pinecone production **characterized by** comprising the following steps:
- Preparation of a stone pine tree biological sample and extraction of DNA from said sample;
- Genotyping the DNA sample for at least one Single Nucleotide Polymorphisms selected from the sequences SEQ ID NO: 1 to 18 associated with higher pinecone production;
- Determining the stone pine tree's production based on the presence of at least one of said Single Nucleotide Polymorphisms of sequences SEQ ID NO: 1 to 18 associated with higher pinecone production.

2. Method according to the previous claim, wherein the sample is a sample of needles.

3. Single Nucleotide Polymorphisms of sequences SEQ ID NO: 1 to 18 for use as biomarkers of pinecone production in stone pine trees.
